(19) **Europäisches Patentamt**
**European Patent Office**
**Office européen des brevets**

(11) **EP 1 132 045 B1**

(12) **EUROPÄISCHE PATENTSCHRIFT**

(45) Veröffentlichungstag und Bekanntmachung des Hinweises auf die Patenterteilung:
**14.01.2009 Patentblatt 2009/03**

(51) Int Cl.:
***A61B 5/0456*** (2006.01)

(21) Anmeldenummer: **01102937.8**

(22) Anmeldetag: **08.02.2001**

(54) **Signalauswerteverfahren zur Detektion von QRS-Komplexen in Elektrokardiogramm-Signalen**

Signal evaluation method for detecting QRS complexes in electrocardiogram signals

Méthode d'évaluation de signaux pour la détection des complexes QRS dans les signaux d'électrocardiogrammes

(84) Benannte Vertragsstaaten:
**CH DE FR GB LI NL**

(30) Priorität: **10.03.2000 DE 10011733**

(43) Veröffentlichungstag der Anmeldung:
**12.09.2001 Patentblatt 2001/37**

(73) Patentinhaber: **BIOTRONIK GmbH & Co. KG**
**12359 Berlin (DE)**

(72) Erfinder:
• **Köhler, Bert-Uwe,**
**c/o Kastner**
**12103 Berlin (DE)**

• **Orglmeister, Reinhold, Prof. Dr.-Ing.**
**13467 Berlin (DE)**

(74) Vertreter: **Lindner-Vogt, Karin L. et al**
**Biotronik GmbH & Co. KG**
**Corporate Intellectual Properties**
**Woehrmannkehre 1**
**12359 Berlin (DE)**

(56) Entgegenhaltungen:
**US-A- 5 309 917**

• **VARY: "Digitale EKG-Triggerung ohne Multiplikationen" ELEKTRONIK, Nr. 10, 1980, Seiten 61-66, XP002178781**

**Beschreibung**

Die Erfindung betrifft ein Signalauswerteverfahren zur Detektion von QRS-Komplexen in Elektrokardiogramm-(EKG-)-Signalen.

Zum Hintergrund der Erfindung ist festzuhalten, daß die automatische Analyse von EKG-Signalen zur Perfektionierung der Funktionsfähigkeit von Herzschrittmachern und Defibrillatoren eine immer größere Rolle spielt. Solche kardiologischen Geräteimplantate jüngerer Bauart bieten dementsprechend auch die Fähigkeit einer EKG-Analyse. In diesem Zusammenhang hat die Erkennung von QRS-Komplexen und R-Zacken in EKG-Signalen einen überragenden Stellenwert. Diese Bedeutung resultiert aus der vielfältigen Verwendbarkeit der Information über das zeitliche Auftreten des QRS-Komplexes, z.B. bei der Untersuchung der Herzratenvariabilität, bei der Klassifikation und bei der Datenkompression sowie als Basissignal für sekundäre Anwendungen. Überhaupt nicht oder falsch detektierte QRS-Komplexe und R-Zacken sind dabei problematisch im Hinblick auf die Leistungsfähigkeit der auf die Detektion folgenden Verarbeitungs- und Analysestufen.

Einen breiten Überblick über bekannte Signalauswerteverfahren zur Detektion von QRS-Komplexen in EKG-Signalen bietet der Fachaufsatz von Friesen et al. "A Comparison of the Noise Sensitivity on Nine QRS Detection Algorithms" in IEEE Transaction on Biomedical Engineering, Vol. 37, Nr. 1, Januar 1990, Seiten 85 - 98. Die dort vorgestellten Signalauswerte-Algorithmen basieren durchgehend auf einer Auswertung der Amplitude, der ersten Ableitung des Signals sowie dessen zweiter Ableitung. Bei den vorgestellten Algorithmen wird unterschieden zwischen solchen, die eine Analyse der Amplitude und der ersten Ableitung vornehmen, solchen, die nur die erste Ableitung bewerten und solchen, die die erste und zweite Ableitung in Betracht ziehen. Kurz umrissen wird bei allen Algorithmen abgeprüft, ob der jeweilige Signalparameter vorgegebene Schwellen über- oder unterschreitet, wonach bei Auftreten eines solchen.Ereignisses mit einem vorgegebenen Schema das Auftreten weiterer definierter Ereignisse abgeprüft und bei Erfüllung bestimmter Kriterien auf das Vorliegen eines QRS-Komplexes geschlossen wird.

Ein anderer Aspekt bei der Signalauswertung zur Detektion von QRS-Komplexen ist bei der Implementierung solcher Verfahren in implantierten Herzschrittmachern zu beachten. Im Hinblick auf die naturgemäß bei solchen Geräten gegebenen Beschränkungen bei der Energieversorgung und der Rechenkapazität ist darauf zu achten, daß zur Detektion von QRS-Komplexen möglichst einfache Algorithmen mit möglichst wenigen Rechenoperationen auf der Basis von ganzen Zahlen anstatt von reellen Zahlen durchzuführen sind.

Ferner wurden zur QRS-Erkennung Signalverarbeitungsmethoden aus den Gebieten der linearen und nichtlinearen Filterung, der Wavelet-Transformation, der künstlichen neuronalen Netze und der genetischen Algorithmen angewendet. Bei hohen Signal-Stör-Abständen und nicht pathologischen Signalen, also im Falle guter Signalverhältnisse, erzielten diese Auswerteverfahren zuverlässige Ergebnisse. Waren keine solchen Verhältnisse gegeben, konnte die Leistungsfähigkeit der Auswerteverfahren unter Umständen drastisch absinken, was im Hinblick auf eine zuverlässige Funktionsweise eines Schrittmachers natürlich nicht akzeptabel ist.

Ausgehend von den geschilderten Problematiken liegt der Erfindung die Aufgabe zugrunde, ein Signalauswerteverfahren zur Detektion von QRS-Komplexen in EKG-Signalen anzugeben, das mit vergleichsweise geringer Rechenkapazität und auch bei problematischen Signalverhältnissen einsetzbar ist und dabei zuverlässige Detektionsergebnisse liefert.

Diese Aufgabe wird durch die im Patentanspruch 1 angegebenen kennzeichnenden Verfahrensschritte wie folgt gelöst:

- Abtasten des Signals und Umwandlung in diskrete, zeitlich aufeinanderfolgende Signalwerte,
- Bestimmung des Vorzeichens jedes Signalwerts,
- laufendes Abprüfen der Vorzeichen aufeinanderfolgender Signalwerte auf Vorliegen eines Nulldurchgangs zwischen zwei aufeinanderfolgenden Signalwerten,
- Ermittlung der Anzahl der Nulldurchgänge in einem definierten Segment der aufeinanderfolgenden Signalwerte, und
- Vergleichen der ermittelten Nulldurchgangszahl mit einem definierten Schwellenwert, wobei ein Unterschreiten des Schwellenwertes signifikant für das Vorliegen eines QRS-Komplexes in dem definierten Segment des Signalverlaufes ist.

Kernpunkt des erfindungsgemäßen Verfahrens ist die Anwendung einer Nulldurchgangszählung, die auf der Ausnutzung der Morphologie des QRS-Komplexes basiert. Der QRS-Komplex im EKG-Signal ist nämlich durch eine relativ hochamplitudige Oszillation charakterisiert, die den Signalverlauf ausgeprägt von der Null-Linie des Elektrokardiogramms wegführt. Die Frequenz dieser kurzen Oszillation liegt in einem Bereich, in dem andere Signalkomponenten, wie die P- und T-Welle, nur geringen Einfluß ausüben und vorzugsweise durch Vorfilterung - z.B. eine Hoch- oder Bandpaßfilterung - entfernt werden können. Nach der Unterdrückung dieser tieffrequenten Signalkomponenten ergeben sich aufgrund von höherfrequentem Rauschen Signalschwankungen um die Null-Linie, die in dem Bereich, wo kein QRS-Komplex auftritt, das EKG-Signal dominieren. Der QRS-Komplex erscheint dann in diesem Signalkontext als langsame, nur kurz andauernde Schwingung hoher Amplitude. Die Unterscheidung zwischen QRS-Kom-plex und den anderen Signalsegmenten kann daher durch eine Frequenzmessung detektiert werden, die sich aufgrund der erörterten Signaleigenschaften repräsentativ durch die Zahl der Nulldurchgänge pro definiertem Auswertesegment beschreiben läßt. Die Nulldurchgangszählung liefert eine Zahl, die nahezu proportional zur jeweils dominanten Frequenz des Signals ist.

Statt der Vorfilterung der Signalwerte zur Entfernung der P- und T-Welle kann beim erfindungsgemäßen Verfahren der QRS-Komplex auch durch Bestimmen der Dauer oder des Zeitpunktes des signifikanten Ausbleibens von Nulldurchgängen innerhalb des EKG-Signals von der P- und T-Welle unterschieden werden.

Das Verfahren der QRS-Komplex-Erkennung durch Nulldurchgangszählung hat sich als robust gegenüber Rauschstörungen und rechentechnisch einfach realisierbar erwiesen. Insoweit eignet es sich besonders für die Implementierung bei der Realzeit-Analyse von EKG-Signal-Morphologien in Herzschrittmachern.

Die bereits erwähnte Hochpaßfilterung wird vorzugsweise mit einer unteren Durchlaßfrequenz von 18 Hz vorgenommen. Damit können die tieffrequenten Komponenten, wie die P- und T-Welle sowie eine Grundliniendrift unterdrückt werden. Ferner wird der QRS-Komplex dadurch zur Signalkomponente mit der niedrigsten Frequenz, die das Signal während ihres Auftretens dominiert.

Zur Vergrößerung des Signal-Stör-Abstandes kann ferner vorgesehen sein, die Signalwerte vor dem Abprüfen auf Nulldurchgänge und dem Ermitteln der Nulldurchgangszahl unter Beibehaltung ihres Vorzeichens zu quadrieren. Damit werden kleine Signalwerte relativ zu größeren Signalwerten abgeschwächt, was die Detektierbarkeit des QRS-Komplexes weiter verbessert.

Dem gleichen Zweck dient das bevorzugte Verfahrensmerkmal der Addition eines hochfrequenten Überlagerungssignals b(n) zu dem Hochpaßgefilterten und unter Beibehaltung des Vorzeichens quadrierten EKG-Signal. Durch diese Maßnahme wird das EKG-Signal so manipuliert, daß sich außerhalb des QRS-Komplexes eine wesentlich besser vorhersagbare Nulldurchgangszahl einstellt. Insbesondere kann durch eine geeignete Wahl der Amplitude das EKG-Signal so aufbereitet werden, daß die Nulldurchgangszahl außerhalb der QRS-Komplexe gleich der Zahl der Signalwerte in dem jeweils herangezogenen Segment ist. Dies bedeutet, daß zwischen jedem Abtastwert ein Nulldurchgang stattfindet, sofern nicht gerade ein QRS-Signal-Komplex detektiert wird. Dieser Effekt wird verstärkt, wenn darüber hinaus der Hochpaß durch einen Bandpaß, vorzugsweise mit den unteren und oberen Durchlaßfrequenzen 18 Hz bzw. 27 Hz, ersetzt wird. Der Wert der Amplitude des hochfrequenten Überlagerungssignals wird vorzugsweise adaptiv aus einer fließenden Mittelung der Bandpaß-gefilterten und quadrierten Signal-werte über eine definierte Mittelungsperiode bestimmt.

Gemäß einer weiteren bevorzugten Ausführungsform des erfindungsgemäßen Signalauswerteverfahrens wird der für einen QRS-Komplex signifikante Schwellenwert der Nulldurchgangszahl als adaptive Schwelle aus sogenannten Quantilen der Häufigkeitsverteilung der Nulldurchgangszahl selbst variabel eingestellt. Näheres hierzu ist der Beschreibung des Ausführungsbeispiels entnehmbar.

Schließlich ist bei der Detektion des QRS-Komplexes auch der Zeitpunkt dessen R-Zacke in kardiologischer Hinsicht interessant. Dieser Zeitpunkt läßt sich ermitteln durch Bestimmung des Maximums der Bandpaß-gefilterten und quadrierten Signalwerte in einem Suchintervall um den Zeitpunkt, an dem die Nulldurchgangszählung D(n) den Schwellwert unterschreitet. Die Gruppenlaufzeit des Bandpaßfilters muß vom Zeitpunkt des Signalmaximums subtrahiert werden, um den Zeitpunkt der R-Zacke zu erhalten.

Schließlich wird als weiteres Kriterium für das Vorliegen eines Störsignals bzw. eines Nutzsignales aus den Signalwerten eine geschätzte Nutz- und Störsignalstärke bestimmt und daraus eine für das Vorliegen eines Störsignals bzw. Nutzsignals signifikante Detektionsstärke ermittelt.

Im folgenden wird das erfindungsgemäße Verfahren anhand der beigefügten Zeichnungen in einem Ausführungsbeispiel näher erläutert. Es zeigen:

Fig. 1 eine höchst schematische Darstellung des Signalverlaufs eines QRS-Komplexes in einem EKG-Signal,

Fig. 2 ein Strukturschema des erfindungsgemäßen Signalauswerteverfahrens zur Detektion von QRS-Komplexen in EKG-Signalen, und

Fig. 3 typische Signalverläufe, wie sie bei Anwendung des erfindungsgemäßen Signalauswerteverfahrens gemäß Fig. 2 auftreten.

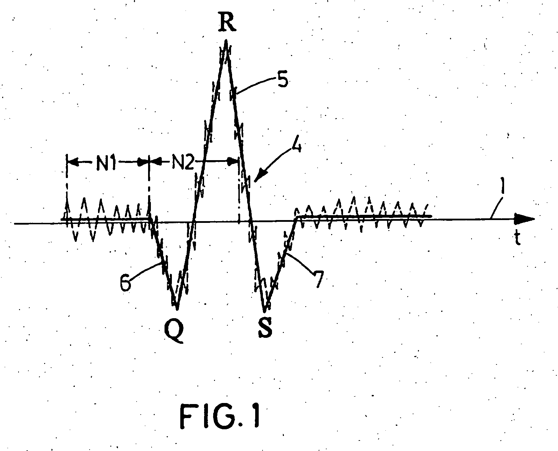

Wie aus Fig. 1 deutlich wird, besteht ein idealisierter QRS-Komplex aus einer relativ hochamplitudigen Oszillation, die das EKG-Signal in der Q-Zacke zuerst in negativer Richtung von der Null-Linie 1 wegführt. Anschließend wird das EKG-Signal in der R-Zacke mit einem steilen Anstieg in den positiven Bereich und einem anschließenden steilen Abfall wiederum in den negativen Bereich unter Ausbildung der S-Zacke übergeführt.

Real ist das EKG-Signal mit einem bestimmten Pegel verrauscht, was in Fig. 1 durch den strichlierten Signalverlauf angedeutet ist. Wird nun dieses verrauschte Signal abgetastet und in diskrete, zeitlich aufeinanderfolgende Signalwerte umgewandelt, kann das Vorzeichen jedes Signalwertes bestimmt und abgeprüft werden, ob zwischen diesen Signalwerten ein Nulldurchgang des EKG-Signals durch die Null-Linie 1 stattgefunden hat. Außerhalb des QRS-Komplexes stellt sich in einem definierten Segment N1 eine hohe Zahl von Nulldurchgängen ein, während bei Erfassung eines Segmentes N2 im QRS-Komplex eine sehr viel niedrigere Anzahl von Nulldurchgängen detektiert wird. Insofern kann die Erfassung der Zahl der Nulldurchgänge zur Erkennung eines QRS-Komplexes herangezogen werden.

Das EKG-Signal wird abgetastet und in diskrete, zeitlich aufeinanderfolgende Signalwerte $x(n)$ umgewandelt. Die Abtastrate beträgt beispielsweise $f_T$ = 360 Hz, d.h., daß das EKG-Signal in eine Folge von 360 Meßwerten pro Sekunde umgewandelt wird.

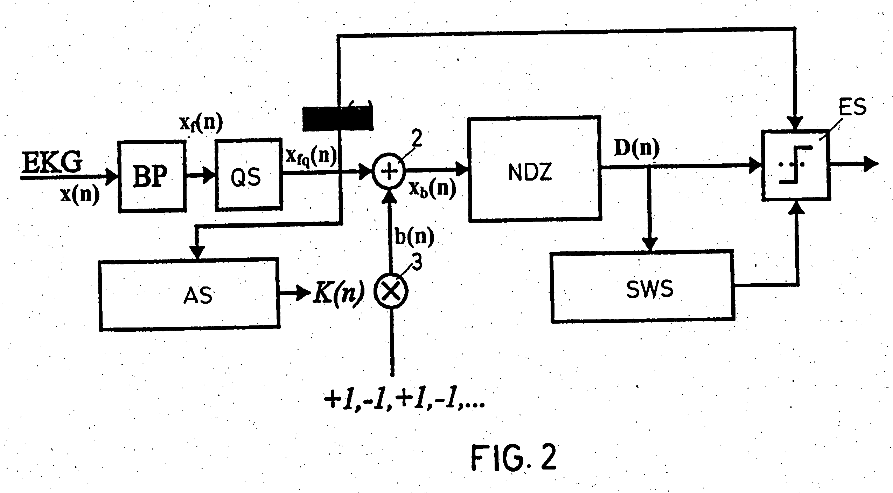

Der detaillierte Ablauf des erfindungsgemäßen Auswerteverfahrens wird nun im folgenden anhand der Fig. 2 näher erläutert. Gemäß diesem Strukturbild wird das abgetastete EKG-Signal $x(n)$ eingangsseitig einer Bandpaßfilterung unterzogen, die der Entfernung aller Signalkomponenten, die nicht zum QRS-Komplex gehören, dient. Dazu zählen die P- und T-Welle sowie hochfrequentere Störungen, die z.B. von der bioelektrischen Muskelaktivität ausgehen. Darüber hinaus wird die Grundliniendrift damit unterdrückt und das EKG auf die Null-Linie 1 geholt. Der eingesetzte Filter BP ist nicht rekursiv, linearphasig und besitzt eine Bandpaßcharakteristik mit den Durchlaßfrequenzen $f_{g1}$ = 18 Hz und $f_{g2}$ = 27 Hz sowie den Sperrgrenzfrequenzen $f_{s1}$ = 2 Hz und $f_{s2}$ = 50 Hz. Die Filterordnung ist N = 200. Die Gruppenlaufzeit des Bandpaßfilters BP entspricht dementsprechend 100 Abtastwerten und muß bei der Bestimmung des Zeitpunktes des QRS-Komplexes berücksichtigt werden. Die Sperrdämpfung des Filters beträgt etwa 80 dB.

Die so erhaltenen Signalwerte $x_f(n)$ werden anschließend in einer Quadrierstufe QS gemäß folgender Beziehung quadriert, wobei das Vorzeichen des jeweiligen Signalwertes beibehalten wird:

$$x_{fq}(n) = \text{sign}[x_f(n)]|x_f(n)|^2$$

Zu dem Bandpaß-gefilterten und quadrierten EKG-Signal wird anschließend in einer Addierstufe 2 eine hochfrequente Sequenz $b(n)$ mit niedriger Amplitude überlagert, die wie folgt zu beschreiben ist:

$$b(n) = (-1)^n K(n)$$

wobei $K(n) > 0$. Durch die Addition dieser Sequenz $b(n)$ wird die Anzahl der Nulldurchgänge pro Segment verändert. Obere Grenze der Nulldurchgangszahl ist die Zahl N der Abtastwerte des Segments. Durch diese Sequenz $b(n)$ wird in den Nicht-QRS-Segmenten die Anzahl der Nulldurchgänge auf diese Maximalanzahl gesteigert, während im QRS-Komplex die (niedrigere) Anzahl der Nulldurchgänge beibehalten wird. Um dieses Ziel zu erreichen wird ein geeigneter Wert für die Koeffizienten $K(n)$ adaptiv aus den Signalwerten $x_{fq}(n)$ geschätzt. Dazu werden fließend die Bandpaß-gefilterten und quadrierten Signalwerte über ein definiertes Mittelungsintervall von P Abtastwerten gemäß folgender Gleichung ermittelt:

$$< |x_{fq}| > (n) = 1/P \cdot \sum_{i=0}^{P-1} | x_{fq} (n-i)|$$

wobei P = 4. (Anzahl Abtastwerte pro Sekunde).

Empirisch ergibt sich für $K(n)$ die Beziehung

$$K(n) = 9 \cdot < |x_{fq}| > (n)$$

Die Mittelungszeit bestimmt im wesentlichen die Adaptionsgeschwindigkeit dieser Schätzung, wobei sowohl zu kurze als auch zu lange Mittelungssegmente die Leistungsfähigkeit des Signalauswerteverfahrens beeinträchtigen können. Während des Auftretens von QRS-Komplexen wird im übrigen die Adaption ausgesetzt, da die Sequenz $b(n)$ die Nulldurchgänge ausschließlich während der Nicht-QRS-Segmente beeinflussen soll.

In Fig. 2 ist der die Ermittlung des Koeffizienten K(n) betreffende Verfahrenskomplex mit AS gekennzeichnet. Die Multiplikation der als eine Art "Flip-Flop-Funktion" in Fig. 2 mit "+1, -1, +1, -1, ..." angedeuteten Grundfunktion mit der Amplitude K(n) ist durch die Multiplikationsstufe 3 angedeutet.

Die vorstehend erörterten Signalwerte werden nun der eigentlichen Nulldurchgangszählung NDZ unterworfen. Grundsätzlich wird die Zählung der Nulldurchgänge pro Segment durchgeführt nach der Beziehung

$$D(n) = \sum_{i=0}^{N-1} d(n-i)$$

wobei N die Segmentlänge angibt. Ferner gilt

$$d(n) = \tfrac{1}{2} |sign[x_b(n)] - sign[x_b(n-1)]|$$

Für d(n) = 1 gilt "Nulldurchgang erkannt", für d(n) = 0 gilt "kein Nulldurchgang erkannt". Auf diese Weise ergibt sich für hohe Frequenzen eine hohe Nulldurchgangszahl pro Segment, für niedrige Frequenzen entsprechend weniger.

In signaltechnischer Hinsicht entspricht die Zählung von Nulldurchgängen im wesentlichen einer Tiefpaß-Filterung, in der Praxis kann die Nulldurchgangszählung mit einem Filter mit einer Rechteck-Impuls-Antwort realisiert werden, d.h. mit der Filter-Impuls-Anwort $a_i = 1$ mit i = 0 ... N-1 ergibt sich die Nulldurchgangszahl D(n). Der Vorteil dieses Filters ergibt sich aus der rechentechnisch günstigen Implementierung mit N-1 Verschiebungsoperationen und einer Rückkoppelung ohne Multiplikation. Die Filterfunktion ist nämlich wie folgt definiert:

$$H(z) = \sum_{i=0}^{N-1} z^{-i} = (1-z^{-(N-1)})/(1-z^{-1})$$

Ein weiterer Vorteil dieser Implementierung besteht darin, daß die Nulldurchgangszahl ausschließlich ganzzahlige Werte annimmt, deren Bereich durch die Segmentlänge N bestimmt wird. Die Eigenschaft kann in der folgenden Entscheiderstufe ES vorteilhaft ausgenutzt werden. Ferner beeinflußt die Filterordnung N wesentlich die Robustheit des Signalauswerteverfahrens gegenüber Störungen. Größere Filterordnungen erhöhen die Robustheit, andererseits können zu lange Filter aufgrund des zu langen Mittelungsintervalls zu falsch-negativen Detektionsfehlern führen ("falschnegativ" bedeutet, daß trotz Vorliegen eines QRS-Komplexes im EKG-Signal dieser nicht erkannt wurde). Im vorliegenden Ausführungsbeispiel wird die Filterordnung N=10 verwendet.

Der für die Erkennung eines QRS-Komplexes signifikante Schwellenwert für die Nulldurchgangszahl wird durch Vergleich mit einer adaptiven Schwelle ermittelt. Diese wird aus dem Mittelwert der 0,1- und 0,5-Quantile der Häufigkeitsverteilung f(m) von D(n) ermittelt. Die statistische Größe "Quantil" wird deswegen verwendet, da sie im Vergleich zu Mittelwert und Varianz eine größere Robustheit gegenüber statistischen Ausreißern aufweist. Im vorliegenden Falle ist ihre Berechnung sehr einfach, da die Signalwerte nur ganzzahlige Werte zwischen $0 \leq D(n) \leq N$ annehmen kann. Die Häufigkeitsverteilung f(m) mit $0 \leq m \leq N$ wird adaptiv in zwei Schritten ermittelt, nämlich

$$f^*_n(m) = (1-\lambda)f_{n-1}(m)$$

und

$$f_n[D(n)] = f^*_n[D(n)] + \lambda$$

wobei ein Gedächtnisfaktor $0 < \lambda < 1$ verwendet wird. Für das am Ende dieser Beschreibung kurz wiedergegebene Zahlenbeispiel wurde dieser Gedächtnisfaktor zu $\lambda = 0{,}01$ gewählt. Aus der Häufigkeitsverteilung können nun die Quantile und in der oben beschriebenen Weise daraus die adaptive Schwelle leicht bestimmt werden. Liegt D(n) unterhalb der Schwelle, ist ein QRS-Komplex erfaßt worden, andernfalls nicht. In Fig. 2 ist der Verfahrensabschnitt der Schwellenwertschätzung mit SWS bezeichnet.

Zur Bestimmung des genauen Zeitpunktes der R-Zacke eines QRS-Komplexes wird im übrigen das Bandpaßgefilterte und quadrierte Signal $x_{fq}(t)$ herangezogen. Zu diesem Zweck wird in einem Suchintervall um den Startpunkt eines QRS-Komplexes das Maximum in diesem Signal gesucht, dessen Auftreten als Zeitpunkt der R-Zacke gesetzt wird.

Parallel zur eigentlichen Detektion von QRS-Komplexen und zur Bestimmung des Zeitpunktes der R-Zacke werden zur Signalbewertung noch zwei Variablen im Auswerteverfahren abgeschätzt, nämlich die Nutzsignalstärke $P_{QRS}$ und die Störsignalstärke $P_{Noise}$. Mit jedem erkannten Ereignis wird eine der beiden Variablen aktualisiert. Bei Detektion eines QRS-Komplexes wird die geschätzte Nutzsignalstärke, andernfalls die geschätzte Störsignalstärke aktualisiert. Dazu werden der Wert $|x_{fq}(t)|_{max}$ in einem geeigneten Intervall um den Zeitpunkt, an dem die Nulldurchgangszahl D(n) den Schwellwert unterschreitet, herangezogen, wobei in der vorliegenden Implementierung jeweils ein exponentielles Fenster gewählt wurde. Dies bedeutet folgende Ableitung für die geschätzte Nutz- bzw. Störsignalstärke:

$$P_{QRS}(i + 1) = (1 - \lambda_{QRS}) \cdot P_{QRS}(i) + \lambda_{QRS} \cdot |x_{fq}(t)_{max}$$

falls QRS-Komplex

$$P_{noise}(i+1) = (1 - \lambda_{Noise)} \cdot P_{Noise}(i) + \lambda_{Noise} \cdot |x_{fq}(t)|_{max} \text{falls Störung.}$$

Die Gedächtnisfaktoren $\lambda$ in den beiden oben stehenden Gleichungen wurden wie folgt gewählt.

$$\lambda_{QRS} = 0{,}5$$

und

$$\lambda_{Noise} = 0{,}01.$$

Aus den geschätzten Signalstärken wird schließlich nach der folgenden Beziehung eine Detektionsstärke berechnet, deren Wert Aufschluß darüber gibt, ob ein an sich als QRS-Komplex zu qualifizierendes Ereignis bei dem Signalauswerteverfahren tatsächlich ein einem QRS-Komplex zuzuordnendes Nutzsignal ist. Die Detektionsstärke wird berechnet durch

$$DS = (|x_{fq}(t)|_{max} - P_{Noise})/(P_{QRS} - P_{Noise})$$

Im vorliegenden Beispiel wird ein erkannter Peak als Störsignalpeak klassifiziert, wenn die Detektionsstärke kleiner als 0,01 ist. In diesem Falle wird die Störsignalstärke aktualisiert. Andernfalls handelt es sich um einen QRS-Komplex, wonach entsprechend die Nutzsignalstärke aktualisiert wird.

Schließlich wird bei der Signalauswertung ein Zeitfenster von 75 ms gesetzt. Werden innerhalb dieses Zeitfensters mehrere QRS-Komplexe detektiert, so wird nur der erste Komplex gewertet und die anderen Komplexe werden ausgeblendet. Diese relativ kurze Refraktärzeit wurde gewählt, um bei falsch-positiven Detektionen eines QRS-Komplexes eine schnelle Wiederaufnahme der normalen Detektion zu gewährleisten und somit falschnegative Erkennungsfehler zu reduzieren.

Das erfindungsgemäße Signalauswerteverfahren, wie es vorstehend ausführlich erörtert wurde, wurde anhand der für Testzwecke kommerziell vertriebenen Datenbank mit der Bezeichnung "MIT/BIH Arrhythmia Data Base" getestet und validiert. Diese Datenbank enthält 48 zweikanalige EKG-Signale mit einer Länge von jeweils etwa 30 Minuten. Diese EKG-Signale sind klassifiziert, so daß die Lage der QRS-Komplexe bekannt ist.

Das Signalauswerteverfahren wurde auf einem Personalcomputer durchgeführt, wobei als Abtastrate eine Frequenz $f_T$ = 360 Hz verwendet wurde. Für die Bewertung der Leistungsfähigkeit des Verfahrens wurden die sogenannte Sensitivität Se und die Spezifität +P gemäß folgender Bedingung ermittelt:

$$Se = TP/(TP + FN) \quad \text{Sensitivität}$$

$$+P = TP/(TP + FP) \quad \text{Spezifität.}$$

wobei mit TP (= True Positive) die Anzahl der korrekt erkannten QRS-Komplexe, FN (= False Negative) die Anzahl der falsch-negativen Erkennungen und FP (= False Positive) die der falsch-positiven Erkennungen herangezogen wurde. Ein QRS-Komplex wurde dabei als richtig erkannt angenommen, wenn er innerhalb eines Zeitfensters von $\pm$ 75 ms um die tatsächliche zeitliche Lage detektiert wurde. Die Ergebnisse dieses Simulationsbeispieles sind in der anhängenden Tabelle 1 aufgelistet. Daraus läßt sich entnehmen, daß für die weit überwiegende Mehrzahl von Datensätzen - den sogenannten "Tapes" - die Sensitivität Se und Spezifität +P weit über 99 % und teilweise glatte 100 % betrugen. Lediglich in einigen wenigen Fällen stark verrauschter Signale, wie bei den Datensätzen (Tape No 105 und 108) waren diese Werte geringer, jedoch nach wie vor so hoch, daß auch dort gute Ergebnisse erhalten wurden.

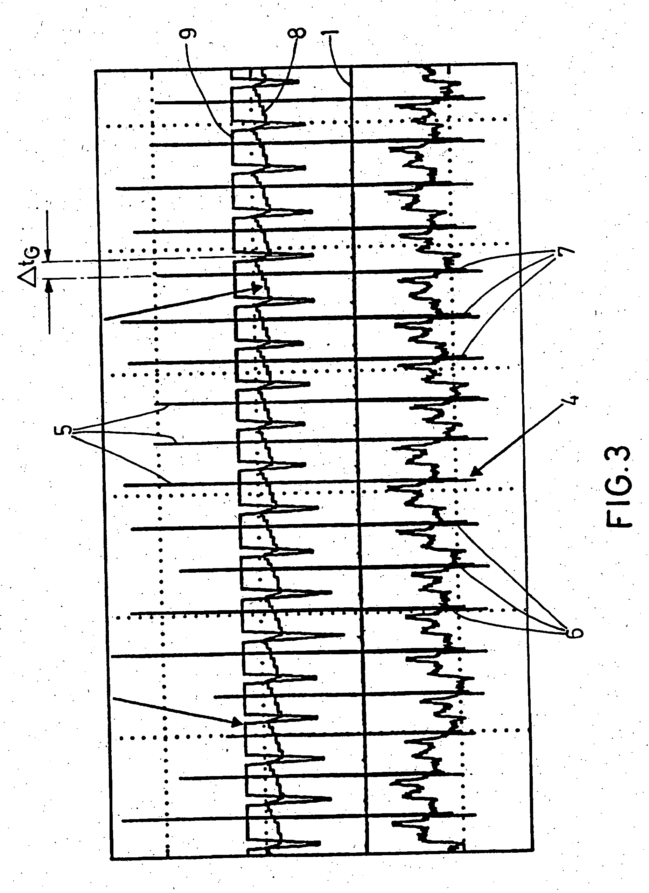

Das Simulationsbeispiel wird im übrigen beispielhaft in Fig. 3 graphisch wiedergegeben. So gibt der Signalverlauf 4 das eigentliche EKG-Signal wieder. Darin sind die R-Zacke 5 deutlich, die unmittelbar daneben liegenden Q- und S-Zacken 6, 7 sind nur andeutungsweise erkennbar.

Eingetragen ist ferner die adaptive Schwelle 8 für die Unterscheidung zwischen QRS- und Nicht-QRS-Segmenten. Darauf basierend gibt die Kurve 9 den Verlauf der Anzahl der Nulldurchgänge der EKG-Signalwerte wieder. Erkennbar bricht die Anzahl der Nulldurchgänge mit einer Verzögerung $\Delta t_G$ nach Auftreten eines QRS-Komplexes ein, die der Gruppenlaufzeit bei der Abtastung und Filterung des EKG-Signals entspricht. Dies äußert sich in den nach unten gerichteten Zacken in der Kurve 9. Synchron damit wird der Schwellenwert 8 nach Auftreten eines QRS-Komplexes adaptiert, wie aus dem sägezahnartigen Verlauf des Schwellenwertes 8 in Fig. 3 deutlich wird.

Tabelle 1: Ergebnisse der QRS-Erkennung mit Nulldurchgangszählung auf der MIT/BIH Arrhythmia Data Base

| Tape No. | Channel | TP | FN | FP | Se (%) | +P (%) |
|---|---|---|---|---|---|---|
| 100 | MLII | 1901 | 1 | 0 | 99,95 | 100,00 |
| 101 | MLII | 1522 | 1 | 8 | 99,93 | 99,48 |
| 102 | V5 | 1808 | 13 | 13 | 99,29 | 99,29 |
| 103 | MLII | 1728 | 1 | 0 | 99,94 | 100,00 |
| 104 | V5 | 1839 | 18 | 18 | 99,03 | 99,03 |
| 105 | MLII | 2151 | 4 | 37 | 99,81 | 98,31 |
| 106 | MLII | 1691 | 5 | 9 | 99,71 | 99,47 |
| 107 | MLII | 1776 | 8 | 7 | 99,55 | 99,61 |
| 108 | MLII | 1448 | 32 | 30 | 97,84 | 97,97 |
| 109 | MLII | 2077 | 22 | 3 | 98,95 | 99,86 |
| 111 | MLII | 1773 | 3 | 12 | 99,83 | 99,33 |
| 112 | MLII | 2110 | 1 | 9 | 99,95 | 99,58 |
| 113 | MLII | 1505 | 1 | 5 | 99,93 | 99,67 |
| 114 | V5 | 1604 | 0 | 6 | 100,00 | 99,63 |
| 115 | MLII | 1636 | 1 | 0 | 99,94 | 100,00 |
| 116 | MLII | 1997 | 20 | 4 | 99,01 | 99,80 |
| 117 | MLII | 1283 | 1 | 2 | 99,92 | 99,84 |
| 118 | MLII | 1916 | 0 | 2 | 100,00 | 99,90 |
| 119 | MLII | 1661 | 0 | 0 | 100,00 | 100,00 |
| 121 | MLII | 1538 | 22 | 33 | 98,59 | 97,90 |
| 122 | MLII | 2053 | 1 | 0 | 99,95 | 100,00 |
| 123 | MLII | 1269 | 0 | 5 | 100,00 | 99,61 |

(fortgesetzt)

| Tape No. | Channel | TP | FN | FP | Se (%) | +P (%) |
|---|---|---|---|---|---|---|
| 124 | MLII | 1365 | 2 | 4 | 99,85 | 99,71 |
| 200 | MLII | 2165 | 3 | 27 | 99,86 | 98,77 |
| 201 | MLII | 1520 | 1 | 84 | 99,93 | 94,76 |
| 202 | MLII | 1870 | 1 | 13 | 99,95 | 99,31 |
| 203 | MLII | 2437 | 44 | 38 | 98,23 | 98,46 |
| 205 | MLII | 2195 | 6 | 0 | 99,73 | 100,00 |
| 207 | MLII | 1586 | 6 | 113 | 99,62 | 93,35 |
| 208 | MLII | 2419 | 18 | 8 | 99,26 | 99,67 |
| 209 | MLII | 2518 | 0 | 6 | 100,00 | 99,76 |
| 210 | MLII | 2200 | 4 | 6 | 99,82 | 99,73 |
| 212 | MLII | 2284 | 1 | 7 | 99,96 | 99,69 |
| 213 | MLII | 2672 | 28 | 26 | 98,96 | 99,04 |
| 214 | MLII | 1876 | 2 | 12 | 99,89 | 99,36 |
| 215 | MLII | 2794 | 1 | 0 | 99,96 | 100,00 |
| 217 | MLII | 1843 | 2 | 8 | 99,89 | 99,57 |
| 219 | MLII | 1773 | 0 | 1 | 100,00 | 99,94 |
| 220 | MLII | 1693 | 1 | 0 | 99,94 | 100,00 |
| 221 | MLII | 2004 | 16 | 17 | 99,21 | 99,16 |
| 222 | MLII | 2116 | 0 | 6 | 100,00 | 99,72 |
| 223 | MLII | 2199 | 0 | 2 | 100,00 | 99,91 |
| 228 | MLII | 1701 | 2 | 55 | 99,88 | 96,87 |
| 230 | MLII | 1859 | 0 | 15 | 100,00 | 99,20 |
| 231 | MLII | 1278 | 0 | 0 | 100,00 | 100,00 |
| 232 | MLII | 1485 | 0 | 22 | 100,00 | 98,54 |
| 233 | MLII | 2550 | 11 | 0 | 99,57 | 100,00 |
| 234 | MLII | 2289 | 2 | 0 | 99,91 | 100,00 |
| Total: | | 90977 | 306 | 673 | 99,66 | 99,27 |

**Patentansprüche**

1. Signalauswerteverfahren zur Detektion von QRS-Komplexen in Elektrokardiogramm-(EKG-)Signalen, **gekennzeichnet durch** folgende Verfahrensschritte:

   - Abtasten des Signals (4) und Umwandlung in diskrete, zeitlich aufeinanderfolgende Signalwerte (x(n)),
   - Bestimmung des Vorzeichens (sign) jedes Signalwerts ($x_f(n)$),
   - laufendes Abprüfen der Vorzeichen (sign) aufeinanderfolgender Signalwerte ($x_f(n)$) auf Vorliegen eines Nulldurchgangs zwischen zwei aufeinanderfolgenden Signalwerten ($x_f(n)$),
   - Ermittlung der Anzahl (D(n)) der Nulldurchgänge in einem definierten Segment (N) der aufeinanderfolgenden Signalwerte ($x_f(n)$), und
   - Vergleichen der ermittelten Nulldurchgängszahl (D(n)) mit einem definierten Schwellenwert, wobei ein Unterschreiten des Schwellenwertes signifikant für das Vorliegen eines QRS-Komplexes (5, 6, 7) in dem definierten Segment des Signalverlaufes (4) ist.

2. Signalauswerteverfahren nach Anspruch 1, **dadurch gekennzeichnet, daß** die EKG-Signalwerte (x(n)) nach dem Abtasten einer Hochpaßfilterung, vorzugsweise einer Bandpaßfilterung (BP) unterworfen werden.

3. Signalauswerteverfahren nach Anspruch 2, **dadurch gekennzeichnet, daß** die untere und obere Durchlaßgrenzfrequenz ($f_{g1}$, $f_{g2}$) des Bandpaßfilters (BP) bei ca. 18 Hz und ca. 27 Hz liegen.

4. Signalauswerteverfahren nach einem der Ansprüche 1 bis 3, **dadurch gekennzeichnet, daß** die Signalwerte ($x_f(n)$) vor dem Abprüfen auf Nulldurchgänge und Ermitteln der Nulldurchgangszahl (D(n)) unter Beibehaltung ihres Vorzeichens quadriert werden.

5. Signalauswerteverfahren mindestens nach Anspruch 2 und 4, **dadurch gekennzeichnet, daß** zu dem Bandpaß-gefilterten und quadrierten EKG-Signal ($x_{fq}(n)$) vor dem Abtasten ein hochfrequentes Überlagerungssignal ($b(n)$) mit im Vergleich zur Amplitude des QRS-Komplexes (5, 6, 7) niedriger Amplitude ($K(n)$) addiert wird.

6. Signalauswerteverfahren nach mindestens nach Anspruch 2, 4 und 5, **dadurch gekennzeichnet, daß** der Wert der Amplitude ($K(n)$) des hochfrequenten Überlagerungssignals ($b(n)$) adaptiv aus einer fließenden Mittelung der bandpaßgefilterten und quadrierten Signalwerte ($x_{fq}(n)$) über eine definierte Mittelungsperiode (P) bestimmt wird.

7. Signalauswerteverfahren nach Anspruch 5 oder 6, **dadurch gekennzeichnet, daß** die Addition des hochfrequenten Überlagerungssignals ($b(n)$) bei Erfassung eines QRS-Komplexes (5, 6, 7) ausgesetzt wird.

8. Signalauswerteverfahren nach einem der Ansprüche 1 bis 7, **dadurch gekennzeichnet, daß** der für einen QRS-Komplex (5, 6, 7) signifikante Schwellenwert der Nulldurchgangszahl als adaptive Schwelle aus Quantilen der Häufigkeitsverteilung ($f(m)$) der Nulldurchgangszahl selbst variabel eingestellt wird.

9. Signalauswerteverfahren nach einem der Ansprüche 1 bis 8, **dadurch gekennzeichnet, daß** zur Ermittlung des Zeitpunktes der R-Zacke (5) des QRS-Komplexes (5, 6, 7) das Maximum der bandpaßgefilterten und quadrierten Signalwerte ($x_{fq}(n)$) in einem Suchintervall um den Startpunkt eines QRS-Komplexes (5, 6, 7) bestimmt wird.

10. Signalauswerteverfahren nach einem der Ansprüche 1 bis 9, **dadurch gekennzeichnet, daß** eine geschätzte Nutzsignalstärke ($P_{QRS}$) und eine geschätzte Störsignalstärke ($P_{Noise}$) aus den Signalwerten ($x_{fq}(n)$) bestimmt und daraus eine für das Vorliegen eines Störsignals bzw. Nutzsignals signifikante Detektionsstärke (DS) vermittelt wird.

**Claims**

1. A signal analysis method for detection of QRS complexes in electrocardiogram (ECG) signals, **characterized by** the following process steps:

   - sampling the signal (4) and converting it into discrete, chronologically successive signal values ($x(n)$),
   - determining the sign of each signal value ($x_f(n)$),
   - ongoing checking of the sign of successive signals values ($x_f(n)$) for the existence of a zero crossing between two successive signals values ($x_f(n)$),
   - determining the number ($D(n)$) of the zero crossings in a defined segment (N) of the successive signal values ($x_f(n)$), and
   - comparing the number of zero crossings ($D(n)$) thereby ascertained with a defined threshold value, such that when the value falls below the threshold value, this is significant for the existence of a QRS complex (5, 6, 7) in the defined segment of the signal curve (4).

2. The signal analysis method according to Claim 1, **characterized in that** the ECG signal values ($x(n)$) are subjected to a high-pass filtering, preferably a bandpass filtering (BP), after sampling.

3. The signal analysis method according to Claim 2, **characterized in that** the lower and upper pass cutoff frequencies ($f_{g1}$, $f_{g2}$) of the bandpass filter (BP) are approximately 18 Hz and approximately 27 Hz.

4. The signal analysis method according to any one of Claims 1 to 3, **characterized in that** the signal values ($x_f(n)$) are squared before being checked for zero crossings and determining the number of zero crossings ($D(n)$) while retaining their sign.

5. The signal analysis method according to at least Claims 2 and 4, **characterized in that** a high-frequency heterodyne signal ($b(n)$) with a low amplitude ($K(n)$) in comparison with the amplitude of the QRS complex (5, 6, 7) is added to the bandpass-filtered and squared ECG signal ($x_{fq}(n)$) before sampling.

6. The signal analysis method according to at least Claims 2, 4 and 5, **characterized in that** the value of the amplitude ($K(n)$) of the high-frequency heterodyne signal ($b(n)$) is determined adaptively from a floating averaging of the bandpass-filtered and squared signal values ($x_{fq}(n)$) over a defined averaging period (P).

7. The signal analysis method according to Claim 5 or 6, **characterized in that** the addition of the high-frequency heterodyne signal (b(n)) is suspended on detection of a QRS complex (5, 6, 7).

8. The signal analysis method according to any one of Claims 1 to 7, **characterized in that** the threshold value of the number of zero crossings which is significant for a QRS complex (5, 6, 7) is set to be variable as an adaptive threshold from quantiles of the frequency distribution (f(m)) of the number of zero crossings itself.

9. The signal analysis method according to any one of Claims 1 to 8, **characterized in that** to ascertain the point in time of the R wave (5) of the QRS complex (5, 6, 7), the maximum of the bandpass-filtered and squared signal values ($x_{fq}(n)$) is determined in a search interval about the starting point of a QRS complex (5, 6, 7).

10. The signal analysis method according to any one of Claims 1 to 9, **characterized in that** an estimated useful signal strength ($P_{QRS}$) and an estimated noise signal strength ($P_{noise}$) is determined from the signal values ($x_{fq}(n)$) and a detection strength (DS) which is significant for the existence of a noise signal and/or useful signal is determined.

## Revendications

1. Procédé d'analyse de signal pour la détection d'ensembles QRS dans des signaux d'électrocardiogramme (EKG), **caractérisé par** les étapes de procédé suivantes :

   - balayage du signal (4) et conversion en valeurs de signal (x(n)) discrètes et consécutives dans le temps,
   - détermination du signe (sign) de chaque valeur du signal ($x_f(n)$),
   - contrôle permanent des signes (sign) de valeurs de signal ($x_f(n)$) consécutives au niveau de la présence d'un passage par zéro entre deux valeurs de signal ($x_f(n)$) consécutives,
   - détermination du nombre (D(n)) des passages par zéro dans un segment (N) défini des valeurs de signal ($x_f(n)$) consécutives, et
   - comparaison du nombre de passages par zéro (D(n)) déterminé avec une valeur seuil définie, un sous-dépassement de la valeur seuil étant significatif de la présence d'un ensemble QRS (5, 6, 7) dans le segment défini de la courbe de signal (4).

2. Procédé d'analyse de signal selon la revendication 1, **caractérisé en ce que** les valeurs de signal d'électrocardiogramme (x(n)) sont soumises après le balayage à un filtrage passe-haut, de préférence un filtrage avec un filtre passe-bande (BP).

3. Procédé d'analyse de signal selon la revendication 2, **caractérisé en ce que** la fréquence limite de passage inférieure et la fréquence limite de passage supérieure ($fg_1$, $fg_2$) du filtre passe-bande (BP) se situent aux environs de 18 Hz et de 27 Hz.

4. Procédé d'analyse de signal selon l'une quelconque des revendications 1 à 3, **caractérisé en ce que** les valeurs de signal ($x_f(n)$) sont élevées au carré avant le contrôle des passages par zéro et la détermination du nombre de passages de zéro (D(n)) en conservant leur signe.

5. Procédé d'analyse de signal au moins selon les revendications 2 et 4, **caractérisé en ce que** avant le balayage, un signal de superposition (b(n)) à haute fréquence avec une amplitude (K(n)) basse par rapport à l'amplitude de l'ensemble QRS (5, 6, 7) est ajouté au signal d'électrocardiogramme ($x_{fq}(n)$) filtré par le filtre passe-bande et élevé au carré.

6. Procédé d'analyse de signal selon au moins les revendications 2, 4 et 5, **caractérisé en ce que** la valeur de l'amplitude (K(n)) du signal de superposition (b(n)) à haute fréquence est déterminée de façon adaptative à partir d'une moyenne courante des valeurs de signal ($x_{fq}(n)$) filtrées avec un filtre passe-bande et élevées au carré sur une période moyenne (P) définie.

7. Procédé d'analyse de signal selon la revendication 5 ou 6, **caractérisé en ce que** l'addition du signal de superposition (b(n)) à haute fréquence est interrompue en cas de saisie d'un ensemble QRS (5, 6, 7).

8. Procédé d'analyse de signal selon l'une quelconque des revendications 1 à 7, **caractérisé en ce que** la valeur seuil, significative d'un ensemble QRS (5, 6, 7), du nombre de passages par zéro en tant que seuil adaptatif est réglée de façon variable à partir de quantiles de répartition de fréquence (f(m)) du nombre de passages par zéro.

9. Procédé d'analyse de signal selon l'une quelconque des revendications 1 à 8, **caractérisé en ce que**, pour déterminer le moment de la pointe R (5) de l'ensemble QRS (5, 6, 7), le maximum des valeurs de signal ($x_{fq}(n)$) filtrées par un filtre passe-bande et élevées au carré est déterminé dans un intervalle de recherche autour du point de départ d'un ensemble QRS (5, 6, 7).

10. Procédé d'analyse de signal selon l'une quelconque des revendications 1 à 9, **caractérisé en ce qu'**une intensité de signal utile ($P_{QRS}$) estimée et une intensité de signal parasite ($P_{Noise}$) estimée sont déterminées à partir des valeurs de signal ($x_{fq}(n)$) et une intensité de détection (DS) significative de la présence d'un signal parasite ou d'un signal utile est déterminée à partir de là.

FIG.1

FIG. 2

EP 1 132 045 B1

FIG.3

EP 1 132 045 B1

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur

- **FRIESEN et al.** A Comparison of the Noise Sensitivity on Nine QRS Detection Algorithms. *IEEE Transaction on Biomedical Engineering,* 01. Januar 1990, vol. 37, 85-98